# EUROPÄISCHE PATENTSCHRIFT

(11) **EP 0 543 284 B1**
(45) Veröffentlichungstag und Bekanntmachung des Hinweises auf die Patenterteilung: **17.09.1997**
(21) Anmeldenummer: 92119405.6
(22) Anmeldetag: 11.11.1992
(51) Int. Cl.: A61M 25/01

(54) **Medizinisches Instrument mit einer Einrichtung zum Führen im Darm**
Medical instrument with device for intestinal insertion
Instrument médical avec dispositif d'insertion intestinale

(30) Priorität: 21.11.1991 DE 4138240
(43) Veröffentlichungstag der Anmeldung: 26.05.1993
(73) Patentinhaber: Krüger, Peter C. Dr., D-23568 Lübeck (DE)
(72) Erfinder: Krüger, Peter C. Dr., D-23568 Lübeck (DE)
(74) Vertreter: Vollmann, Heiko, Dipl.-Ing.

(56) Entgegenhaltungen:
- DE-B- 1 170 586
- US-A- 3 895 637
- US-A- 4 447 227

## Beschreibung

Die Erfindung betrifft ein medizinisches Instrument mit Einrichtung zum Führen im Darm, insbesondere ein Endoskop, mit den im Oberbegriff des Anspruches 1 angegebenen Merkmalen.

Beim Führen von medizinischen Instrumenten, insbesondere Endoskopen, im Darm ergeben sich häufig Probleme. Bei Instrumenten ohne besondere Einrichtung zum Führen kann dieses nur durch von außerhalb des Körpers aufbringbare Druckkräfte, sei es unmittelbar auf das Instrument oder mittelbar durch Druck auf entsprechende Körperstellen, vorgeschoben werden. Aufgrund der zahlreichen Darmwindungen ist ein solches Führen ohne Hilfseinrichtung, insbesondere dann, wenn in weiter innen liegende Darmteile vorgestoßen werden soll, problematisch, zeitraubend und für den Patienten häufig auch mit Schmerzen verbunden. Schließlich besteht die Gefahr, daß der Darm durch das Vorschieben des Instrumentes perforiert oder in sonstiger Weise verletzt wird.

Es ist bekannt, ein solches medizinisches Instrument schrittweise unter zeitweiser Anlage an die Darmwand einzuführen. Hierzu ist am distalen Instrumentenende eine Hilfseinrichtung vorgesehen, die mindestens zwei an die Darmwand zum Zwecke der Abstützung anlegbare Teile aufweist, die zueinander in Achsrichtung des Instrumentes verschiebbar sind. Auf diese Weise wird ein Vortrieb des Instrumentes innerhalb des Körpers am distalen Instrumentenende erreicht, so daß der nachfolgende Instrumententeil praktisch in den Darm nachgezogen wird. Eine solche Einrichtung ist aus DE 36 31 352 A1 bekannt.

Prinzipiell sind derartige Hilfseinrichtungen zum Führen des Instrumentes im Darm zweckmäßig. In der Praxis haben sie jedoch auch Nachteile. Denn dieses raupenartige Fortbewegen innerhalb des Darmes ist recht zeitaufwendig und beinhaltet eine latente Verletzungsgefahr der Darmwand. Auch sind derartige Führeinrichtungen technisch aufwendig und somit anfällig. Schließlich treten konstruktionsbedingt häufig Probleme bei der Reinigung und Desinfektion auf, da solche Hilfseinrichtungen aus Kostengründen nicht als Einwegartikel zu fertigen sind. Dies ist insbesondere wegen der wachsenden Aidsproblematik von besonderer Bedeutung.

Aus DE-A-11 70 586 ist eine Darmsonde bekannt, die sich nach Einführung in den Darm selbsttätig vorwärts bewegt. Es handelt sich dabei um einen Saugschlauch mit druckgesteuerter Vortriebseinrichtung. Die Vortriebseinrichtung weist ein Dreikammersystem auf, das über nur eine Druckleitung steuerbar ist. Die Steuerung erfolgt über diese Leitung sowohl mit Unterdruck als auch mit Überdruck, wodurch praktisch eine zweite Leitung ersetzt wird. Die dort beschriebene Darmsonde ist zum Führen eines Endoskopes im Darm weder vorgesehen noch geeignet.

Hiervon ausgehend liegt der Erfindung die Aufgabe zugrunde, ein gattungsgemäßes medizinisches Instrument mit Einrichtung zum Führen im Darm zu schaffen, das unter weitgehender Vermeidung der vorgenannten Nachteile ein einfaches und sicheres Führen innerhalb des Darmes erlaubt.

Diese Aufgabe wird gemäß der Erfindung durch die in Anspruch 1 aufgeführten Merkmale gelöst.

Demgemäß sieht die Erfindung ein medizinisches Instrument, insbesondere ein Endoskop mit Einrichtung zum Führen im Darm vor, das eine durch Druck- bzw. Unterdruckbeaufschlagung an die Darmwand anlegbare Kammer aufweist, die druckgesteuert in Achsrichtung des Instrumentes kontrahierbar und expandierbar ist. Es handelt sich dabei um eine einzige Kammer, die proximalseitig fest und distalseitig verschiebbar am Instrument angeordnet ist.

Die Erfindung schafft somit ein völlig neues Bewegungsprinzip. Mit dem erfindungsgemäßen Instrument wird zunächst ein Teil der das Instrument umgebenden Darmwand durch die Führungseinrichtung auf dem Instrument zusammengeschoben und dann die Führungseinrichtung unter Ausnutzung der darmeigenen Trägheit schnell vorgeschoben. Die Darmwand wird dabei quasi auf das Instrument geschoben, das nach mehreren solcher Zyklen manuell nachgeführt wird. Hierdurch ist ein sehr schnelles und zugleich gefahrloses und schmerzfreies Durchwandern des Instrumentes durch den Darm möglich. Die auf die Darmwand wirkenden Kräfte sind minimal. Die Kontraktion und damit das Aufschieben der Darmwand auf dem Instrument erfolgt definiert, wodurch das Verletzungsrisiko minimiert wird. Da das Instrument nur eine einzige an die Darmwand anlegbare Kammer aufweist, ist es im Vergleich zu Mehrkammersystemen deutlich einfacher herzustellen, zu reinigen und zu steuern. Mit dieser Kammer wird das Instrument zeitweise zur Anlage an die Darmwand gebracht, die dann zusammen mit der Kammer in Achsrichtung des Instrumentes kontrahiert wird. Anschließend erfolgt eine Druckentlastung, wodurch die Kammer axial expandiert und dadurch weiter in das Innere des Darmes in Richtung Dünndarm wandert. Die Führungseinrichtung ist so am Endoskop angeordnet, daß das proximale Ende der Einrichtung fest und das distale Ende verschiebbar auf dem Instrument gehalten ist.

Um das Instrument, insbesondere die Einrichtung zum Führen, an die Darmwand zu bringen, kann sie entweder in (radialer) Richtung auf die Darmwand expandiert werden oder aber die Darmwand in Richtung auf die Einrichtung gezogen werden. Für erstere Lösung kann beispielsweise eine ringförmige Manschetten quasi auf dem Instrument aufgeschoben wird. Die Manschetten sind beweglich auf dem Instrument gelagert.

Die Kontraktion in radialer Richtung erfolgt durch Kollaps der flexiblen Manschetten, in axialer Richtung hingegen ist eine zusätzliche Kraft erforderlich, die beispielsweise durch einen elastischen Schlauch als Feder gebildet sein kann, der am distalen Manschettenende einerseits sowie am distalen Instrumentenende andererseits festgelegt ist. Damit beim Rückfedern in axialer Richtung die Darmwand nicht wieder mit den Manschetten mitwandert, sondern in der aufgeschobenen Lage auf dem Instrument verharrt, ist es erforderlich, daß die Manschetten zunächst durckentlastet und dann, nach dem Ablösen von der Darmwand distalwärts bewegt werden. Hierzu ist eine Arretiervorrichtung vorgesehen, die die Manschetten zumindest so lange axial festhält, bis die Ablösung von der Darmwand erfolgt ist. Erst dann erfolgt ein Nachvorneschieben der Manschetten unter Ausnutzung der Eigenträgheit der Darmwand, die in aufgeschobener Stellung auf dem Instrument verharrt. Eine solche Arretiervorrichtung kann beispielsweise durch einen Ring in Verbindung mit einem diesen steuernden Druckschlauch gebildet sein, so daß bei Druckbeaufschlagung der Schlauch radial gedehnt und damit der Ring auf dem Instrument arretiert wird. Bei Druckabfall hingegen kann dieser Ring auf dem Instrument gleiten.

Bei einer bevorzugten Ausführung, bei der innerhalb der Kammer ein federkraftbeaufschlagter Schlauch angeordnet ist, der über seinen Umfang verteilt Öffnungen zur Kammer aufweist, wobei die druckwirksame Fläche dieser Öffnungen kleiner ist als diejenige am distalen Schlauchende, kann das Arretieren des distalen Schlauchendes druckgesteuert über die den Schlauch beaufschlagende Druckleitung erfolgen. Hierzu ist distalseitig der Manschetten ein den federkraftbeaufschlagten Schlauch zum Instrument hin abschließender elastischer Schlauch vorgesehen, der bei plötzlichem Druckabfall spontan kollabiert und das distale Manschettenende auf dem Instrument festlegt. Da die druckwirksame Fläche der zur Kammer weisenden Schlauchöffnungen kleiner als die druckwirksame distale Stirnfläche am Schlauchende ist, wird bei erneuter Druckbeaufschlagung zunächst die Arretierung gelöst, so daß die Manschetten distalwärts expandieren, bevor bei weiterem Druckanstieg auch eine radiale Expansion zur Anlage an die Darmwand erfolgt.

Statt durch Druck kann die Einrichtung auch durch entsprechenden Unterdruck an die Darmwand zur Anlage gebracht werden. Hierzu weist die Einrichtung über ihre gesamte Länge Umfangsöffnungen auf, die zum Ansaugen der Darmwand vorgesehen sind. Eine solche mit Öffnungen versehene Kammer kann beispielsweise durch einen Faltenbalg oder einen Federschlauch gebildet sein, wobei die Öffnungen nach Möglichkeit tiefgründig liegen, d.h. instrumentenseitig angeordnet sein sollten, damit sich die Darmwand möglichst gleichmäßig um die Kammer anlegt und somit eine punktuelle Druckbelastung der Darmwand vermieden wird.

Beim Einsatz eines Faltenbalges erfolgt die Kontraktion durch Anlegen von Unterdruck. Diese kann durch einen über den Faltenbalg gezogenen flexiblen, nicht dehnbaren Schlauch noch erhöht werden, der zur Übertragung des kammerseitigen Druckniveaus ebenfalls Ansaugöffnungen aufweisen muß.

Die Erfindung ist nachfolgend anhand von in den Zeichnungen dargestellten Ausführungsbeispielen näher erläutert. Es zeigen in stark schematisierter und vereinfachter Darstellung:
- Figur 1: einen distalen Instrumententeil mit darauf angeordneter Einrichtung zum Führen im Darm,
- Figur 2: die Einrichtung gemäß Figur 1 in einer anderen Arbeitsstellung,
- Figur 3: die Einrichtung nach Figur 1 in einer dritten Arbeitsposition,
- Figur 4: eine weitere Ausführungsform einer mit Manschetten arbeitenden Einrichtung in Darstellung nach Figur 1,
- Figur 5: eine mit Federschlauch arbeitende Einrichtung in Darstellung nach Figur 1,
- Figur 6: die Einrichtung nach Figur 5 in einer zweiten Arbeitsstellung,
- Figur 7: eine mittels Faltenbalg arbeitende Einrichtung in Darstellung nach Figur 1,
- Figur 8: eine andere Ausführungsform einer mit Faltenbalg arbeitenden Einrichtung in Darstellung nach Figur 1 und
- Figur 9: eine Einrichtung beim rektalen Einführen in Darstellung nach Figur 1.

In den Figuren ist mit 1 der distale Endbereich eines medizinischen Instrumentes, beispielsweise eines Endoskopes bezeichnet. Auf diesem Instrument ist eine Einrichtung zum Führen angeordnet, die proximalseitig (in den Figuren auf der linken Seite) fest mit dem Instrument verbunden und distalseitig verschiebbar auf dem Instrument gehalten ist.

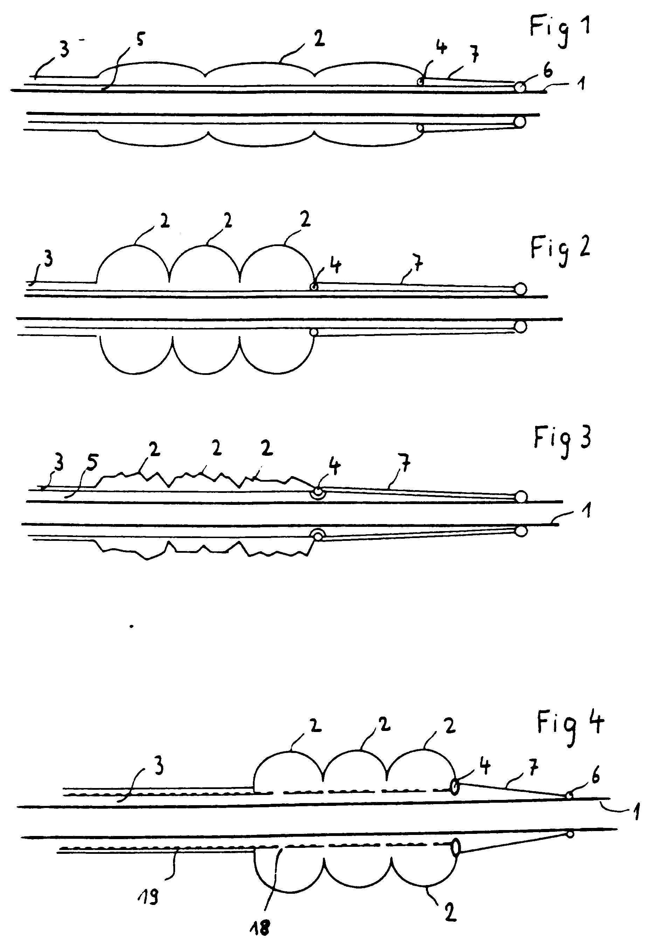

Die anhand der Figuren 1 bis 3 dargestellte Einrichtung besteht aus drei hintereinander auf dem Instrument angeordneten flexiblen und nicht dehnbaren Manschetten 2, die kommunizierend miteinander verbunden sind und die über eine Druckleitung 3 druckbeaufschlagbar sind. Die Druckleitung 3 ist zum proximalen Instrumentenende geführt. Die Manschetten 2 sind distal mit einem Ring 4 verbunden, der Teil einer Arretiervorrichtung ist. Der Ring 4 liegt nicht unmittelbar auf dem Instrument 1 auf, sondern ist durch einen das Instrument 1 in diesem Bereich umgebenden Druckschlauch 5 vom Instrument 1 getrennt, der ebenfalls Teil der Arretiervorrichtung ist. Durch Druckbeaufschlagung des Schlauches 5 wird der Ring 4 axial auf dem Instrument festgelegt und somit arretiert. Bei Druckentlastung hingegen ist der Ring 4 beweglich.

Die Führeinrichtung wird an ihrem distalen Ende durch einen Ring 6 abgeschlossen, der fest auf dem Instrument 1 gehalten ist. Zwischen diesem Ring 6 und dem Ring 4 ist ein elastischer Schlauch 7 als Zugfederelement angeordnet.

Die Funktion der anhand der Figuren 1 bis 3 dargestellten Führeinrichtung ergibt sich wie folgt:
In der in Figur 1 dargestellten vollständig entlasteten Stellung, in der die Leitungen 3 und 5 druckentlastet und der Schlauch 7 entspannt ist, wird das Instrument 1 mit der Einrichtung zunächst ein Stück in den Darm eingeführt. Sodann wird die Leitung 3 druckbeaufschlagt, wodurch sich die Manschetten radial dehnen. Sie legen sich dabei an die (nicht dargestellte) Darmwand an und nehmen diese bei ihrer in Achsrichtung des Instrumentes erfolgenden Kontraktion mit, so daß ein Abschnitt der Darmwand auf das Instrument 1 quasi aufgeschoben wird. Die Einrichtung nimmt dann die in Figur 2 dargestellte Stellung ein. Sodann wird die Leitung 5 druckbeaufschlagt, wodurch der Ring 4 auf dem Instrument 1 festgelegt wird. Erst nachdem der Ring 4 arretiert ist, werden die Manschetten 2 über die Leitung 3 druckentlastet, so daß sich diese radial kollabieren und von der zusammengeschobenen Darmwand lösen. Es ergibt sich dann die in Figur 3 dargestellte Stellung. Danach wird die Leitung 5 druckentlastet, wodurch die Arretierung des Ringes 4 aufgeschoben wird und aufgrund der Spannkraft des elastischen Schlauches 7 der Ring 4 zusammen mit den daran befestigten Manschetten 2 wieder distalwärts in die in Figur 1 dargestellte Position gezogen wird. Es liegt dann ein Teil des Darms aufgeschoben auf dem Instrument. Nach mehreren solcher vorbechsriebenen Arbeitszyklen kann dann das Instrument um ein erhebliches Stück weiter eingeführt werden.

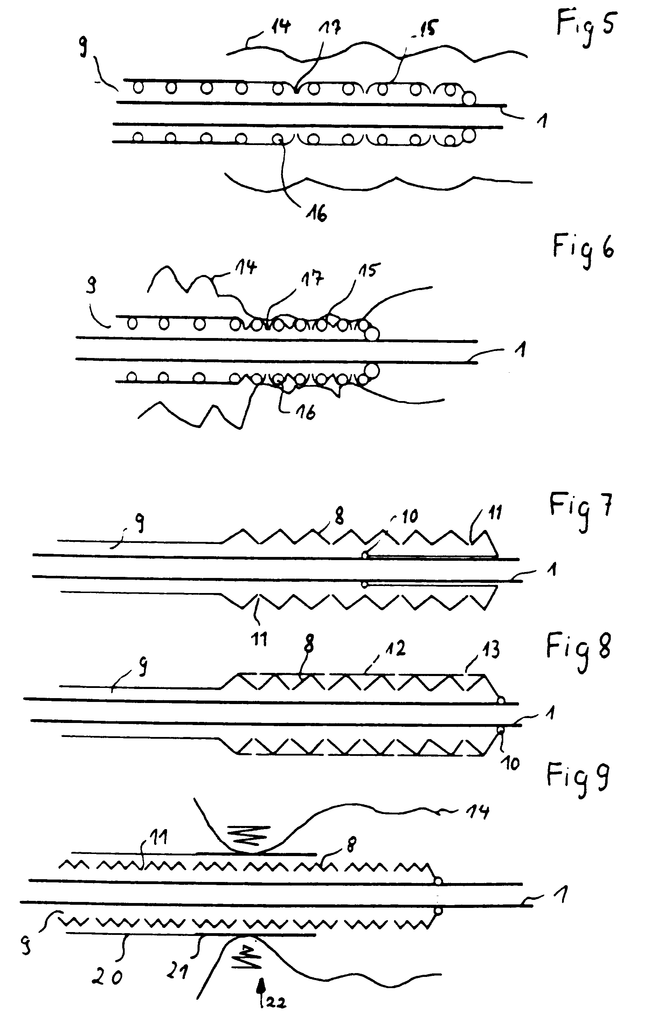

Bei der in Figur 4 dargestellten Ausführung erfolgt die Druckversorgung ausschließlich durch die Leitung 3. Die Manschetten 2 sitzen auf einem das Instrument 1 umgebenden axialfederelastischen Schlauch 19, der über Öffnungen 18 kommunizierend mit den Manschetten 2 verbunden ist. Die Druckbeaufschlagung der Manschetten 2 erfolgt in gleicher Weise wie anhand der Figuren 1 und 2 vorbeschrieben. Dann, wenn der Darm durch die axial kontrahierten Manschetten 2 auf dem Instrument 1 aufgeschoben ist, wird durch eine plötzliche Druckentlastung in der Leitung 3 der Kontakt der Manschetten 2 zur Darmwand unterbrochen. Durch diese Druckentlastung kollabieren nicht nur die Manschetten 2, sondern auch der elastische Schlauch 7 zwischen den Ringen 4 und 6, wodurch der Ring 4 adhäsiv auf dem Instrument 1 festgelegt wird. Nachdem die Manschetten 2 genügend weit kollabiert sind, wird der Schlauch 3 wiederum druckbeaufschlagt. Da die druckwirksame Fläche der Öffnungen 18 kleiner ist als die druckwirksame Fläche am distalen Schlauchende, die durch einen Teil des Rings 4 sowie den Schlauch 7 gebildet wird, erfolgt bei erneuter Druckbeaufschlagung zunächst eine Distalwärtsbewegung des Ringes 4 und dann erst eine radiale Expansion der Manschetten 2.

Die anhand der Figuren 5 bis 9 dargestellten Einrichtungen arbeiten mit Unterdruck, d.h. die Darmwand wird mittels Unterdruck an das Instrument zur Anlage gebracht und dann auf diesem zusammengeschoben, wonach unter Ausnutzung der darmeigenen Trägheit nach kurzer Druckbeaufschlagung schnell vorgeschoben wird.

Bei einer weiteren Ausführung entsprechend den Figuren 5 und 6 ist ein Abschnitt der Darmwand mit 14 gekennzeichnet. Die Einrichtung besteht hier aus einem proximal auf dem Instrument festgelegten und distal verschiebbar angeordneten flexiblen Schlauch 15, der durch eine schraubenlinienförmig angeordnete Feder 16 in distaler Richtung, also in axialer Expansionsrichtung federkraftbeaufschlagt ist. Der Schlauch 15 ist über eine Druckleitung 9 angeschlossen, er weist Öffnungen 17 auf, über die die Darmwand 14 angesaugt bzw. wieder abgestoßen wird.

Die Arbeitsweise dieser Einführeinrichtung ist anhand der Figuren 5 und 6 deutlich erkennbar. In einem ersten Arbeitsschritt wird der Schlauch 15 an Unterdruck gelegt, so daß sich die umgebende Darmwand am Umfang des Schlauches 15 festsaugt und schließlich, sobald ein dichtes System zwischen Schlauch und Darmwand gebildet ist, durch den Unterdruck der Schlauch 15 in seinem Endbereich in Achsrichtung des Instrumentes kontrahiert. Bei dieser Ausführung liegen die Öffnungen 17 möglichst weit innen, um eine Verletzung der Darmwand durch Druckbeaufschlagung zu verhindern.

Wenn der Schlauch 15 schließlich seine vollständig kontrahierte Stellung erreicht hat, wird dieser mit einem erhöhten Druck beaufschlagt, so daß der aufgeschobene Darmabschnitt 14 vom Schlauch 15 abhebt und der Schlauch 15 aufgrund der Federkraft der Schraubenfeder 16 zum distalen Instrumentenende hin vorrückt. Dann beginnt ein neuer Zyklus.

Figur 7 zeigt ein Instrument 1 mit einem Faltenbalg 8, der über eine Leitung 9, die zum proximalen Instrumentenende geführt ist, mit Druck bzw. Unterdruck beaufschlagbar ist. Der Faltenbalg 8 ist proximalseitig am Instrument 1 festgelegt, sein distales Ende ist verschiebbar auf dem Instrument 1 angeordnet, er ist mittels einer am Instrument festgelegten Dichtung 10 gegenüber diesem abgedichtet. Diese Einrichtung arbeitet in gleicher Weise wie die anhand der Figuren 5 und 6 vorbeschrieben.

Die Ausführung nach Figur 8 unterscheidet sich von der nach Figur 7 zum einen in der Anordnung der Dichtung 10 und zum anderen durch einen äußeren Hüllschlauch 12. Die Dichtung 10 liegt bei dieser Ausführung am distalen Ende des Faltenbalgs und gleitend dem Instrument an. Der Hüllschlauch 12 umgibt den Faltenbalg 8 vollständig, er weist Öffnungen 13 auf, durch die das in der Leitung 9 anstehende Druckniveau weitergeleitet wird. Der Hüllschlauch 12 bewirkt eine Kraftverstärkung beim Kontrahieren des Faltenbalgs 8. Aufgrund der glatteren Außenoberfläche ist zudem ein schonenderes Aufschieben des Darms sowie ein leichtes rektales Einführen möglich.

Bei den vorbeschriebenen Ausführungen nach den Figuren 5 bis 9 können die Öffnungen 11 bzw. 17 so gestaltet sein, daß sie eine gewisse Ventilfunktion ausüben, d.h., daß sie bevorzugt eine Strömung von außen nach innen zulassen und in Gegenrichtung querschnittsverengend wirken.

In Figur 9 ist ein Instrument 1 mit einer Einführeinrichtung dargestellt, wie sie beispielhaft anhand der Figuren 5 bis 9 beschrieben ist, also eine Einrichtung, bei der die Darmwand 14 mittels Unterdruck anlegbar ist. Bei diesen Einrichtungen, die Ansaugöffnungen über die gesamte Länge und über ihren Umfang verteilt aufweisen, ist es erforderlich, beim rektalen Einführen dafür zu sorgen, daß der noch nicht im Darm befindliche Teil der Einrichtung nach außen hin abgedichtet wird, damit sich in dem innerhalb des Darms befindlichen Abschnitt des Instrumentes der erforderliche Unterdruck aufbauen kann. Hierzu ist ein Folienschlauch 20 vorgesehen, der sich von einer Einführhilfe in Form eines Rohres 21 bis zum proximalen Ende der Einrichtung erstreckt und diesen Bereich nach außen hin abdichtet. Das Rohr 21 ist im Bereich des Schließmuskels 22 eingeführt, um radiale Belastung und Verletzung des Darmes zu vermeiden.

## Patentansprüche

1. Medizinisches Instrument (1) mit Einrichtung zum Führen im Darm, insbesondere Endoskop, mit einer durch Druck- bzw. Unterdruckbeaufschlagung an die Darmwand (14) anlegbaren Kammer (2; 8; 15), die druckgesteuert in Achsrichtung des Instrumentes (1) kontrahierbar und expandierbar ist, dadurch gekennzeichnet, daß nur eine einzige Kammer (2; 8; 15) vorgesehen ist, die proximalseitig fest und distalseitig verschiebbar am Instrument (1) angeordnet ist.

2. Medizinisches Instrument nach Anspruch 1, dadurch gekennzeichnet, daß die axiale Expansion der Kammer (2; 8; 15) durch Federkraft erfolgt.

3. Medizinisches Instrument nach Anspruch 1 oder 2, dadurch gekennzeichnet, daß die Kammer (2; 8; 15) an ihrem proximalen Ende mittelbar über einen Schlauch (9) oder unmittelbar fest mit dem Instrument (1) verbunden ist.

4. Medizinisches Instrument nach einem der vorhergehenden Ansprüche, dadurch gekennzeichnet, daß die Kammer (2) aus mehreren kommunizierend miteinander verbundenen flexiblen Manschetten besteht, die in Achsrichtung des Instrumentes distalwärts feder- und/oder druckkraftbeaufschlagt sind.

5. Medizinisches Instrument nach Anspruch 4, dadurch gekennzeichnet, daß die Manschetten (2) distal mit einem axialelastischen Schlauch (7) verbunden sind, der distalseitig am Instrument (1) festgelegt ist und daß das distale Manschettenende mittels einer Arretiervorrichtung (4;5;7) am Instrument (1) festlegbar ist.

6. Medizinisches Instrument nach Anspruch 5, dadurch gekennzeichnet, daß die Arretiervorrichtung (4;5;7) einen durch Druckbeaufschlagung am Instrument (1) festlegbaren Ring (4) aufweist.

7. Medizinisches Instrument nach einem der Ansprüche 1 bis 4, dadurch gekennzeichnet, daß innerhalb der Kammer (2) ein federkraftbeaufschlagter Schlauch (19) angeordnet ist, der über seinen Umfang verteilt Öffnungen (18) zur Kammer (2) aufweist, wobei die druckwirksame Fläche der Öffnungen kleiner ist als diejenige am distalen Schlauchende.

8. Medizinisches Instrument nach einem der Ansprüche 1 bis 3, dadurch gekennzeichnet, daß die Kammer (8; 15) in dem zur Anlage an die Darmwand (14) bestimmten Bereich über ihre gesamte Länge Öffnungen (11; 17) aufweist, die zum Ansaugen der Darmwand (14) vorgesehen sind.

9. Medizinisches Instrument nach einem der Ansprüche 1 bis 3 oder 8, dadurch gekennzeichnet, daß die Kammer (8) aus einem elastischen Faltenbalg gebildet ist.

10. Medizinisches Instrument nach Anspruch 9, dadurch gekennzeichnet, daß die Öffnungen (11) nahe dem Innendurchmesser des Faltenbalgs (8) angeordnet sind.

11. Medizinisches Instrument nach einem der Ansprüche 1 bis 3 oder 8, dadurch gekennzeichnet, daß die Kammer (15) als Federschlauch ausgebildet ist, wobei sich die Feder (16) schraubenlinienförmig längs des Schlauches erstreckt und die Federkraft distalwärts gerichtet ist.

12. Medizinisches Instrument nach Anspruch 9 oder 10, dadurch gekennzeichnet, daß der Faltenbalg (8) von einem elastischen Schlauch (12) überzogen ist, der Ansaugöffnungen (13) für die Darmwand aufweist.

13. Medizinisches Instrument nach einem der vorhergehenden Ansprüche, gekennzeichnet durch eine Einführhilfe, im wesentlichen bestehend aus einem beim Einführen die Kammer (2; 8; 15) umgebenden Stützkörper (21) sowie einer sich daran anschließenden bis zum proximalen Ende der Einführeinrichtung reichenden flexiblen Dichthülle (20).

## Claims

1. A medical instrument (1), with a device for guiding within the intestine, in particular an endoscope, with a chamber (2; 8; 15) which can be rested on the intestinal wall (14) by way of pressure or vacuum impinging, said chamber being pressure-controllably contractable and expandable in the axial direction, characterized in that only a single chamber (2; 8; 15) is provided which is rigidly arranged on the instrument at the proximal side and is displaceably arranged on the instrument at the distal side.

2. A medical instrument according to claim 1, characterized in that the axial expansion of the chamber (2;8;15) is effected by spring force.

3. A medical instrument according to claim 1 or 2, characterized in that the chamber (2; 8; 15) at its proximal end is connected to the instrument (1) indirectly via a hose (9) or is directly rigidly connected to said instrument.

4. A medical instrument according to one of the preceding claims, characterized in that the chamber (2) comprises several flexible collars communicatingly connected to one another which are impinged distally in the axial direction of the instrument by spring force or pressure force.

5. A medical instrument according to claim 4, characterized in that the collars (2) are connected distally to an axial-elastic hose (7) which on the distal side is fastened to the instrument, and that the distal collar end can be fastened to the instrument (1) by way of a retention device (4;5;7).

6. A medical instrument according to claim 5, characterized in that the retention device (4;5;7) comprises a ring (4) which can be fastened to the instrument (1) by way of pressure impinging.

7. A medical instrument according to one of claims 1 to 4, characterized in that within the chamber (2) there is arranged a hose (19) impinged by spring force which comprises, about its circumference, openings (18) to the chamber (2), wherein the pressure effective area of the openings is smaller than that on the distal hose end.

8. A medical instrument according to one of claims 1 to 3, characterized in that the chamber (8; 15), in the region destined to rest on the intestinal wall, comprises openings (11; 17) over its whole length, said openings being provided for aspirating the intestine.

9. A medical instrument according to one of claims 1 to 3, characterized in that the chamber (8) is formed from an elastic bellows.

10. A medical instrument according to claim 9, characterized in that the openings (11) are arranged near to the inner diameter of the bellows (8).

11. A medical instrument according to one of claims 1 to 3, characterized in that the chamber (15) is formed as a spring hose, wherein the spring (16) extends helically shaped along the hose and the spring force is directed distally.

12. A medical instrument according to claim 9 or 10, characterized in that the bellows (8) is coated by an elastic hose which comprises aspiration openings (13) for the intestinal wall.

13. A medical instrument according to one of the preceding claims, characterized by an introduction aid, essentially composed of a support body (21) surrounding the chamber (2; 8; 15) on introduction, as well as, connected to said support body, a flexible sealing cover (20) reaching to the proximal end of the introduction device.

## Revendications

1. Instrument médical (1) muni d'un dispositif pour le diriger dans l'intestin, en particulier endoscope, comportant une chambre (2; 8; 15) qui peut être appliquée sur la paroi (14) de l'intestin en étant soumise à une pression ou à une dépression, et qui peut se contracter et se dilater dans la direction de l'axe de l'instrument (1) par une commande de la pression, caractérisé en ce qu'il n'est prévu qu'une seule chambre (2; 8; 15), qui est disposée sur l'instrument (1) en position fixe, côté proximal, et en étant déplaçable, côté distal.

2. Instrument médical selon la revendication 1, caractérisé en ce que la dilatation axiale de la chambre (2; 8; 15) se fait sous l'action de la force d'un ressort.

3. Instrument médical selon la revendication 1 ou 2, caractérisé en ce que la chambre (2; 8; 15), à son extrémité proximale, est reliée indirectement à l' instrument (1) par l' intermédiaire d'un tuyau (9) ou y est fixée directement.

4. Instrument médical selon l'une des revendications précédentes, caractérisé en ce que la chambre (2) est constituée de plusieurs manchettes flexibles reliées l'une à l'autre en communiquant entre elles, qui sont sollicitées par la force d'un ressort et/ou une force de pression dans la direction de l'axe de l'instrument, vers le côté distal.

5. Instrument médical selon la revendication 4, caractérisé en ce que les manchettes (2) sont reliées, côté distal, à un tuyau axialement élastique (7) qui, côté distal est disposé immobile sur l'instrument (1), et en ce que l'extrémité distale des manchettes peut être immobilisée sur l'instrument (1) au moyen d'un dispositif de blocage (4; 5; 7).

6. Instrument médical selon la revendication 5, caractérisé en ce que le dispositif de blocage (4; 5; 7) comporte une bague (4) pouvant être immobilisée sur l'instrument (1) sous la sollicitation d'une pression.

7. Instrument médical selon l'une des revendications 1 à 4, caractérisé en ce qu'à l'intérieur de la chambre (2), est disposé un tuyau (19) sollicité par la force d'un ressort, tuyau qui présente, réparties sur sa circonférence, des ouvertures (18) débouchant dans la chambre (2), la surface agissant en pression des ouvertures étant plus petite que celle sur l'extrémité distale du tuyau.

8. Instrument médical selon l'une des revendications 1 à 3, caractérisé en ce que la chambre (8; 15), dans la région destinée à s'appliquer sur la paroi (14) de l'intestin, présente, sur sa longueur entière, des ouvertures (11; 17) qui sont prévues pour exercer une aspiration sur la paroi (14) de l'intestin.

9. Instrument médical selon l'une des revendications 1 à 3 ou 8, caractérisé en ce que la chambre (8) est formée par un soufflet élastique.

10. Instrument médical selon la revendication 9, caractérisé en ce que les ouvertures (11) sont situées à proximité du diamètre intérieur du soufflet (8).

11. Instrument médical selon l'une des revendications 1 à 3 ou 8, caractérisé en ce que la chambre (15) est réalisée sous la forme d'un tuyau à ressort, le ressort (16) s'étendant sous une forme hélicoïdale le long du tuyau et la force du ressort étant dirigée vers le côté distal.

12. Instrument médical selon la revendication 9 ou 10, caractérisé en ce que le soufflet (8) est recouvert par un tuyau élastique (12), qui comporte des ouvertures d'aspiration (13) pour la paroi de l'intestin.

13. Instrument médical selon l'une des revendications précédentes, caractérisé par un accessoire facilitant l'introduction, constitué essentiellement d'un corps d'appui (21) entourant la chambre (2; 8; 15) lors de l'introduction, ainsi que d'une gaine d'étanchéité flexible (20) se raccordant à ce corps d'appui et parvenant jusqu'à l'extrémité proximale du dispositif d'introduction.
